# EUROPEAN PATENT APPLICATION

(11) **EP 1 693 450 A1**
(43) Date of publication of application: **23.08.2006**
(21) Application number: 04818954.2
(22) Date of filing: 18.11.2004
(51) Int. Cl.: C12N 15/09, C12Q 1/68, G01N 33/50, G01N 33/15

(54) **METHOD OF EVALUATING RNAi ACTIVITY AND miRNA ACTIVITY**

(30) Priority: 21.11.2003 JP 2003392304
(71) Applicant: Bio-Think Tank Co., Ltd., Tokyo 113-0033 (JP)
(72) Inventor: NAITO, Yuki, Tokyo 1020082 (JP); TEI, Kumiko, Tokyo 1130023 (JP); ZENNO, Shuhei, Tokyo 1130032 (JP); SAIGO, Kaoru, Tokyo 1680063 (JP)
(74) Representative: Jones, Elizabeth Louise
(86) International application number: PCT/JP2004/017182
(87) International publication number: WO 2005/049821

(57) **Abstract**

The present invention provides methods whereby an RNAi activity and a miRNA activity can be evaluated simply and conveniently. The RNAi activity or the miRNA activity is evaluated by supplying a target-expressing molecule, which is a polynucleotide containing at least a target sequence and an expression regulatory region regulating the expression of an RNA containing the target sequence, and a subject nucleic acid molecule to be evaluated on whether or not it has RNAi activity on the RNA containing the target sequence into an expression system in which the target-expressing molecule can express the RNA containing the target sequence.

## Description

### CROSS REFERENCE TO RELATED APPLICATIONS

This application claims the benefit of priority to a Japanese Patent Application No. 2003-392304, filed on November 21, 2003 (Heisei 15), the entire contents of which are incorporated herein by reference.

### TECHNICAL FIELD

The present invention relates to methods for evaluating RNAi activity and miRNA activity, and more particularly to methods whereby RNAi activity and miRNA activity can be simply and conveniently evaluated. It should be noted that "RNAi" described herein is also referred to as RNA interference. In addition, "miRNA" described herein is also referred to as microRNA.

### BACKGROUND ART

RNA interference is the phenomenon in which double-stranded RNA (hereinafter referred to as "dsRNA"), composed of sense RNA and antisense RNA homologous to a certain region of a gene whose function is to be inhibited, interferes with and disrupts the homologous part of an mRNA, the transcript of the target gene. The phenomenon was first discovered in 1998 by experiments with nematode (Fire, A. et al., Potent and specific gene interference by double-stranded RNA in Caenorhabditis elegans, Nature, 391: 806-811, 1998). The RNAi method whereby expression of a gene of interest is inhibited using this phenomenon has been a focus of attention. However, it has been difficult to use the RNAi method in mammals, because, when long dsRNAs of about 30 or more base pairs are introduced into cells, an interferon response is induced, thereby causing cell death by apoptosis.

Meanwhile, it was shown that RNAi can occur in early mouse embryos and cultured mammalian cells, supplying evidence that the mechanism of RNAi induction itself is present in mammals as well. It has now been shown that short double-stranded RNAs (short interfering RNA; hereinafter referred to as "siRNA") that are about 21 to 23 base pairs long can induce RNAi without exhibiting cytotoxicity in mammalian cell systems; it is becoming possible to use the RNAi method in mammals (Zamore, P. D. et al., RNAi: double-stranded RNA direct the ATP-dependent cleavage of mRNA at 21 to 23 nucleotide intervals. Cell, 101: 25-33 2000; and Elbashir, S. M. et al., Duplexes of 21-nucleotide RNAs mediate RNA interference in cultured mammalian cells: Nature, 411: 494-498, 2001).

Further, it has recently been suggested that noncoding RNAs of about 20 nucleotides long are involved in translation suppression or mRNA degradation. Such RNAs of about 20 nucleotides long are called miRNAs (microRNAs) (Grishok, A. et al., The rde-1 gene, RNA amplification in dsRNA interference regulate expression of the small temporal RNAs that control C. elegans developmental timing. Cell, 110: 513-520, 2002; and Rhoades, M. W. et al., Prediction of plant microRNA targets, Cell, 110: 513-520, 2002).

### DISCLOSURE OF THE INVENTION

The RNAi method is a technique promising for a wide variety of applications. However, while in Drosophila and nematodes dsRNAs and siRNAs homologous to a certain region of genes in almost all sequences exhibit RNA interference, in mammals, 70 to 80% of siRNAs (of 21 nucleotides) randomly selected do not exhibit the RNA interference effect. This has caused a big problem in gene function analysis using the RNAi method in mammals. Furthermore, when an endogenous gene is targeted, since the expression level, stability, etc. of mRNA vary depending on the gene, in some cases it is difficult to evaluate the efficacy of siRNA. Moreover, much of the mechanism of miRNAs remains unknown; thus methods for simply and conveniently evaluating miRNA activity are desired.

Under the above-mentioned circumstances, the problem for the present invention is to provide methods whereby RNAi activity and miRNA activity can be simply and conveniently evaluated.

The inventors of the present application have assiduously studied to solve the above-mentioned problem, and found a simple and convenient method of introducing a molecule to be evaluated and a polynucleotide containing a target sequence into an expression system to evaluate whether or not the molecule to be evaluated exerts an RNAi activity or a miRNA activity on the target sequence. Thus, the present invention provides the methods for evaluating RNAi activity and miRNA activity listed below.
(1) A method for evaluating RNAi activity, including the steps of:
   supplying a target-expressing molecule, which is a polynucleotide containing at least a target sequence and an expression regulatory region which regulates expression of an RNA containing the target sequence, and
   a subject nucleic acid molecule to be evaluated on whether or not the subject nucleic acid molecule has an RNAi activity on the RNA containing the target sequence
   into an expression system in which the target-expressing molecule is capable of expressing the RNA containing the target sequence; and
   detecting whether or not the RNA containing the target sequence has been cleaved.
(2) The evaluation method for RNAi activity in (1), in which the target-expressing molecule contains, both upstream and downstream of the target sequence, a PCR primer annealing region containing a sequence to which a PCR primer can anneal, the method including the steps of:
   quantifying the RNA containing the target sequence generated by supplying the target-expressing molecule and the subject nucleic acid molecule into the expression system by RT-PCR using a pair of primers annealing to the sequence in the PCR primer annealing region; and
   detecting whether or not the RNA containing the target sequence has been cleaved by comparing with the case in which the subject nucleic acid molecule is not supplied into the expression system.
(3) The method for evaluating RNAi activity of (2), in which an intron is flanked within at least one of the PCR primer annealing regions present both upstream and downstream of the target sequence, and the expression system is an expression system capable of splicing an RNA.
(4) The method for evaluating RNAi activity of (1), in which the target-expressing molecule contains a sequence encoding an expression product detectable as a reporter at a location whereby the reporter is transcribed as an mRNA integrated with the target sequence, the method including a step of detecting the expression of the reporter to detect whether or not the RNA has been cleaved.
(5) The method for evaluating RNAi activity of any one of (1) to (4), in which the expression system is a cell-free expression system or a cell expression system.
(6) The method for evaluating RNAi activity of any one of (1) to (5), including supplying into the expression system into which the target-expressing molecule and the subject nucleic acid molecule are supplied a control-supplying molecule which contains a control sequence containing a sequence to be transcribed into an mRNA that is not cleaved by the subject nucleic acid molecule and encodes a detectable expression product.
(7) The method for evaluating RNAi activity of any one of (1) to (5), in which a control sequence which contains a sequence to be transcribed into an mRNA that is not cleaved by the subject nucleic acid molecule and encodes a detectable expression product is integrated into the target-expressing molecule.
(8) The method for evaluating RNAi activity of any one of (1) to (7), in which the target sequence contains a nucleotide sequence different from the nucleotide sequence that the subject nucleic acid molecule contains.
(9) A method for evaluating miRNA activity, including the steps of:
   supplying a target-expressing molecule, which is a polynucleotide containing:
      at least a target sequence, an expression regulatory region regulating expression of an RNA containing the target sequence, and a sequence encoding an expression product detectable as a reporter at a location whereby the reporter is to be transcribed as an mRNA integrated with the target sequence; and
      a subject nucleic acid molecule to be evaluated on whether or not the subject nucleic acid molecule has an RNAi activity on an RNA containing the target sequence
      into an expression system in which the target-expressing molecule is capable of expressing an RNA containing the target sequence; and
      detecting whether or not the RNA containing the target sequence has been cleaved.
(10) The method for evaluating miRNA activity of (9), in which the expression system is a cell-free expression system or a cell expression system.
(11) The method for evaluating miRNA activity of (9) or (10), including a step of supplying into the expression system into which the target-expressing molecule and the subject nucleic acid molecule are supplied a control-supplying molecule which contains a control sequence containing a sequence whose expression is not suppressed by the subject nucleic acid molecule and encodes a detectable expression product.
(12) The method for evaluating miRNA activity of (11), in which the control-supplying molecule is integrated into the target-expressing molecule.
(13) The method for evaluating miRNA activity of any one of (9) to (12), in which the target sequence contains a nucleotide sequenced different from the nucleotide sequence that the subject nucleic acid molecule contains.

In accordance with the present invention, there are provided methods whereby RNAi activities and miRNA activities can be simply and conveniently evaluated. The methods according to the present invention are excellent particularly in that they allow simple and convenient evaluations of RNAi activity or miRNA activity by targeting arbitrary sequences, regardless of the origin or type of the sequences, such as endogenous gene-derived sequences or artificial sequences. Being simple and convenient, the evaluation methods according to the present invention allow large numbers of tests to be conducted in a short period of time.

### BRIEF DESCRIPTION OF THE DRAWINGS

FIG. 1 shows an example of a target-expressing molecule.
FIG. 2 shows an example a target-expressing molecule: PCR fragment 1.
FIG. 3 shows an example of PCR fragment 2.
FIG. 4 shows a correlation between the number of PCR cycles and the amount of PCR products.
FIG. 5 shows an example of a target-expressing molecule into which a puromycin resistance gene ("puro") is integrated as a reporter.
FIG. 6 shows an example of a target-expressing molecule into which EGFP ("EGFP") is integrated as a reporter.
FIG. 7 shows an example of a target-expressing molecule into which a firefly luciferase gene ("F. Luc") is integrated as a reporter.
FIG. 8 shows the structure of the target expression vector pTREC.
FIG. 9 shows a result of a PCR when one of the primers is not designed to flank an intron in Example 1. 2. (2).
FIG. 10 shows a result of a PCR when one of the primers is designed to flank a intron in Example 1. 2. (2).
FIG. 11 shows the sequence and the structure of the siRNA: siVIM-35.
FIG. 12 shows the sequence and the structure of the siRNA: siVIM-812.
FIG. 13 shows the sequence and the structure of the siRNA: siControl.
FIG. 14 shows RNAi activities of siVIlM-812 and siVIM-35 on the luciferase gene.
FIG. 15 shows RNAi activities of siControl, siVIM-812, and siVIM-35 on vimentin.
FIG. 16 shows results of an antibody staining.

The reference letters in the drawings are as follows:
"pro/enh": Promoter/enhancer
"intron": Intron
"PAR": Primer annealing region
"target": Target sequence
"MCS": Multi-cloning site
"poly A": Poly A signal sequence
"Kozak": Kozak sequence
"Kozak+ATG": Kozak sequence and ATG sequence
"neo": neomycin resistance gene
"puro": puromycin resistance gene
"F. Luc": Firefly luciferase gene
"R. Luc": Renilla luciferase gene

### DETAILED DESCRIPTION OF THE PREFERRED EMBODIMENTS

Preferred embodiments of the present invention are described below by referring to best modes in the following order:
1. Method for evaluating RNAi activity;
2. Method for evaluating miRNA activity;
3. Method for searching for RNA interference target nucleotide sequences;
4. Method for designing the nucleotide sequences of polynucleotides triggering RNA Interference;
5. Method for preparing double-stranded polynucleotides;
6. Method for suppressing gene expression.

Many standard laboratory manuals are available on basic molecular biological techniques, including Basic Methods in Molecular Biology (1986); Sambrook et al., Molecular Cloning: A Laboratory Manual, the 2nd edition, Cold Spring Harbor Laboratory Press, Cold Spring Harbor, N.Y.(1989) Spring Harbor, N.Y. (1989); " Saibo Kogaku Handbook (Cell Engineering Handbook)," edited by Toshio Kuroki, published by Yodosha, 1992; "Shin Idenshi Kogaku Handbook (New Genetic Engineering Handbook)", edited by Muramatsu M. et al., published by Yodosha (Tokyo), 1991, etc., the contents of all of which are herein incorporated by reference. In addition, a nucleotide sequence as used herein is represented using a sense strand, i.e., an mRNA sequence as the reference.

### 1. Method for evaluating RNAi activity

In the method for evaluating an RNAi activity according to the present invention, a target-expressing molecule and a subject nucleic acid molecule are supplied into an expression system capable of expressing RNA, and whether or not RNA expressed from the target-expressing molecule has been cleaved by the subject nucleic acid molecule is detected.

A target-expressing molecule is a molecule for expressing RNA that has an RNAi target sequence (hereinafter referred to as a "target sequence"). The target-expressing molecule may be any polynucleotide that can provide RNA of a target sequence for an expression system. A preferred target-expressing molecule is a DNA from the viewpoints of stability, operativity, etc. and, more specifically, an expression vector etc. can be suitably used. Many expression vectors are available including those commercially available. When using an expression vector to express RNA, the expression vector can be appropriately selected, depending on, for example, the kind of cultured cell system or conditions for the cell-free system, etc. To allow insertion of a target sequence or other predetermined sequences, the target-expressing molecule preferably has a multi-cloning site (sometimes abbreviated as "MCS"). Vectors to be used are illustratively pCI-neo etc.

To supply a target sequence into an expression system, the target-expressing molecule contains at least a target sequence and an expression regulatory region regulating expression of an RNA containing this target sequence. The target sequence is arbitrary, and a person who makes evaluations can arbitrarily select a sequence from a desired gene etc. To give an example, the sequence identified by the "3. Method for searching for RNA interference target nucleotide sequences" described below or some other methods can be selected as a target sequence. Whether the selected sequence can actually be a target of RNAi will be examined in an experimental system. The target-expressing molecule contains an expression regulatory region for expressing RNA containing the target sequence. The expression regulatory region is composed, for example, of a promoter, an enhancer, and/or a poly A region, etc. The promoter and the enhancer, etc. are appropriately selected depending on the conditions of the vector, the host, etc. Additionally, the target-expressing molecule may contain other sequences, such as a terminator, which can be integrated into the vector.

The promoter is not limited as long as it is suitable for cultured cells to be used for gene expression. For example, when using animal cells as host cells, the promoter is illustratively the SRα promoter, SV40 promoter, HIV-LTR promoter, cytomegalovirus (CMV) promoter, HSV-TK promoter, or CAG promoter, etc.

The subject nucleic acid molecule is a nucleic acid molecule which has a sequence serving as a subject of the evaluation of an RNAi activity. The nucleic acid molecule is a molecule composed mainly of nucleic acids; a nucleic acid molecule may be an RNA-DNA hybrid molecule, a chimeric molecule, or a denatured molecule of these. The denatured molecule includes a molecule in which a duplex is not formed perfectly by matched base pairs, as well as a molecule in which a part of the nucleotides are modified. To be more specific, for example, an siRNA candidate molecule which serves as a subject for the evaluation of the degree of RNAi activity on the aforementioned target sequence, or a nucleic acid molecule expressing the siRNA candidate molecule, may be used. Various molecules, such as molecules discovered by chance, molecules designed in the expectation that they will exhibit RNAi activity, can also serve as the candidate molecules.

The method for designing a sequence with high probability of having RNAi activity includes, for example, the "4. 4. Method for designing the nucleotide sequences of polynucleotides triggering RNA Interference" described later. Thus, the evaluation method according to the present invention is suitably used also as a technique to confirm simply and conveniently in an experimental system the RNAi activity of a molecule with high probability of having an siRNA activity which has been obtained according to the "4. Method for designing the nucleotide sequences of polynucleotides triggering RNA interference" and the "5. Method for preparing double-stranded polynucleotides" described later.

The subject nucleic acid molecule is a molecule for supplying the RNAi candidate molecule into the expression system and may be in the form of an siRNA itself, or a vector expressing a molecule in the form of the siRNA.

The target-expressing molecule and the subject nucleic acid molecule can also be supplied into an expression system in which the RNA containing the target sequence can be expressed from the target-expressing molecule. The expression system capable of expressing the RNA may be a cell system or a cell-free system, but it should at least meet the conditions to allow transcription of mRNA. For this reason, the expression system includes materials and enzymes etc. for synthesizing an RNA molecule. In the cell system, the target-expressing molecule can be expressed within suitable cultured cells. The technique for supplying the target-expressing molecule and the subject nucleic acid molecule into the cultured cells includes the conventional techniques such as transfection etc. which allows introduction of molecules into the cells. The cell-free system is, for example, a system in which a cell extract, ribonucleic acids, an RNA polymerase, and ATP, etc. are added to a predetermined buffer etc. extracellularly, e.g. in a test tube.

When using a host cell for the cell expression system, examples of the host cells include insect cells, such as the Drosophila S2 and Spodoptera Sf9; animal cells, such as CHO, COS, HeLa, C127, NIH3T3, BHK and HEK293, Bowes melanoma cells, and hematopoietic cells; and plant cells.

The mRNA containing the target sequence is transcribed from the target-expressing molecule incorporated into the expression system. Then, the RNA containing the target sequence is cleaved if the subject nucleic acid molecule, introduced together with the target-expressing molecule into the expression system, interferes with the subject sequence; and if the molecule does not interfere, the RNA is not cleaved. Therefore, by detecting whether the mRNA has been cleaved or not, it is possible to evaluate whether or not -- or to what degree -- the subject nucleic acid molecule has the RNAi activity. Methods for detecting whether or not RNA has been cleaved include methods for qualitatively or quantitatively measuring RNA expression; a method where a certain reporter capable of being transcribed together with the target sequence is integrated into the target-expressing molecule and the reporter is qualitatively or quantitatively measured; and the like. Methods for specific detections and their suitable forms will be explained later in detail.

When a candidate molecule having an RNAi activity which targets an endogenous gene is directly administered to cells or an organism that has the endogenous gene, it is difficult to evaluate the effect of RNAi if the expression level of the endogenous gene is low, or if the stability of the gene products is low. In addition, in a cultured cell system with low transfection efficiency, even if the molecule introduced has an RNAi activity, the effect of suppression of the endogenous gene by siRNAs can not be clearly demonstrated. Further, when measuring RNA expression level by, for example, reverse transcribed-PCR (RT-PCR), the protocol should be extensively adjusted in terms of combinations of primers, the conditions of PCR cycles, and the like, according to the targeted endogenous gene. For example, suppose there are 10,000 endogenous genes to be targeted, the combinations of 10,000 primers and the conditions of PCR cycles need to be extensively examined.

In contrast, the evaluation method according to the present invention is a simple and convenient method which allows measurement of an RNAi activity by using a simple and convenient experimental system, such as cloning, gene expression system, or the like. According to the evaluation method of the present invention, there is provided simplicity and convenience in which the RNAi activity can be evaluated for many target sequences using a fixed protocol except that a target sequence of interest should be modified. In addition, since an established experimental system, such as an expression vector-host system, can be used as the base, even if the in vivo expression level of the targeted endogenous gene is low, the RNAi activity can be appropriately evaluated. Moreover, even when an expression product including a target sequence would be degraded in vivo at an early stage intrinsically, a cell system or a cell-free system in which the cause of such a problem is eliminated can be prepared comparatively easily according to the method of the present invention. Furthermore, in the evaluation method according to the present invention, siRNAs and a vector can be simultaneously introduced into an expression system. This enables an assay even in a system with a low efficiency of transfection. RNAi is being considered for various uses, such as the gene knockout technology, medical applications, etc. RNAi is the field that has a huge potential for development in the future. The evaluation method according to the present invention greatly contributes to the development of RNAi-related technologies.

Next, preferred embodiments of the evaluation method according to the present invention will be more specifically described hereinbelow.

### 1-1 PCR method

An embodiment in which whether or not an RNA including a target sequence has been cleaved is detected by using RT-PCR is explained. In this embodiment, a cDNA corresponding to a transcribed RNA is amplified by PCR. By detecting the amplified DNA, whether or not the RNA has been cleaved is determined.

In the PCR method of the present invention, a target-expressing molecule includes, both upstream and downstream of the target sequence, a PCR primer annealing region containing a sequence to which the PCR primer can anneal. The sequence of the primer is not particularly limited and is appropriately designed according to the conditions of the PCR etc. In a more preferred embodiment, the target-expressing molecule is designed such that an intron is flanked within at least one of the PCR primer annealing regions present both upstream and downstream of the target sequence. To "be flanked" means not being linked to an end of a PCR primer annealing region but being integrated such that the PCR primer annealing region is divided into two by the intron. By having an intron flanked within the PCR primer annealing region, an amplification of DNA using the expression vector as a template can be prevented. As a result, PCR is performed based on an RNA that has been transcribed from the expression vector and has undergone splicing, i.e., the RNA with introns removed, enabling a more accurate evaluation.

Introns can be found in a genomic DNA of eukaryotes. The intron to be used in the present invention is not limited as long as it has a sequence which does not code for amino acids and is removed in an mRNA splicing. There are many examples of introns, some of which are illustratively β-globin derived introns, IgG-derived introns, etc.

In an embodiment in which an intron is flanked within the PCR primer annealing region, an expression system which allows an RNA splicing is used. For example, as the expression system allowing the RNA splicing, an eukaryotic expression vector-host system in which the RNA splicing has been confirmed to take place may be used. Alternatively, for a cell-free system, a system including at least a spliceosome required for splicing may be constructed. In addition, a mixed solution containing a homogenate of eukaryotic cells may be used as the cell-free system.

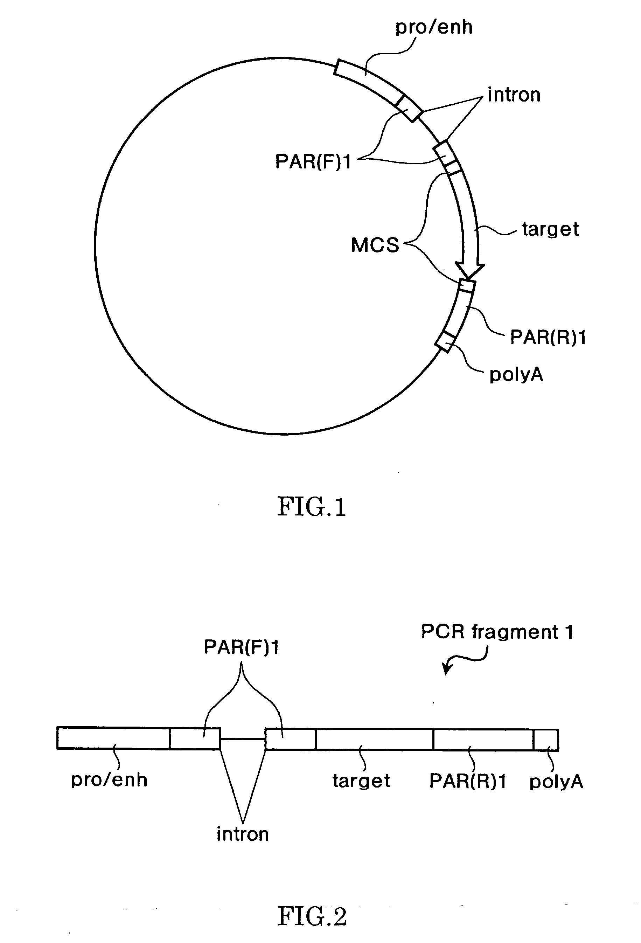

FIG. 1 shows a design example where an expression vector is used as the target-expressing molecule. A promoter and enhancer ("pro/enh") is integrated as an expression regulatory region. A primer annealing region ("PAR(F)1") for a forward primer in which an intron ("intron") is flanked is inserted downstream of the expression regulatory region. A multiple cloning site ("MCS") is located downstream of the primer annealing region ("PAR (F)1") for a forward primer; a target sequence ("target") of interest is inserted using the multiple cloning site ("MCS"). Further, downstream of the target sequence ("target"), a primer annealing region ("PAR(R)1") for a reverse primer is inserted.

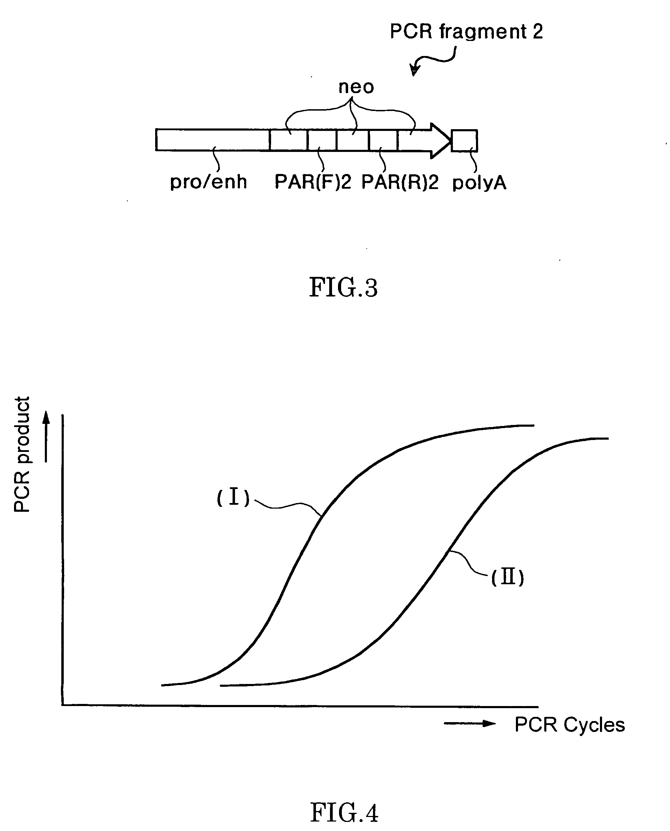

FIGs. 2 and 3 show combinations of PCR base fragments as the target-expressing molecules. The combinations of FIGs. 2 and 3, being used in the PCR method, also have an internal standard control for normalizing detected signals (hereinafter referred to also as "internal control").

FIG. 2 shows a PCR fragment 1 ("PCR fragment 1"), a target-expressing molecule containing a target sequence of 23 nucleotides. FIG. 3 shows a PCR fragment 2 ("PCR fragment 2") used as a control for relative quantification of the expression level of the target sequence site. Use of the control enables corrections of errors among samples, such as variations in transfection efficiency and variations in RNA yield etc. The PCR fragments 1 and 2 are composed of double-stranded DNA. The PCR fragment 1 has a promoter and enhancer ("pro/enh") and, downstream thereof, a primer annealing region ("PAR(F) 1") for a forward primer. Inside the primer annealing region ("PAR(F) 1") an intron ("intron") is flanked so that the PCR fragment 1 itself does not function as a PCR template. It should be noted that, although in the example shown in FIG. 2 the intron is flanked within PAR(F)1, it may be flanked within PAR(R) 1 as well. A target sequence ("target") is inserted downstream of the primer annealing region ("PAR (F)1") for the forward primer. Downstream of the target sequence ("target"), a primer annealing region ("PAR(R) 1") for a reverse primer is inserted. Further downstream of PAR (R) 1, a poly A signal ("polyA") sequence is located. In PCR fragment 2 shown in FIG.3, a neomycin resistance gene ("neo"), used as a control in a relative quantification, is integrated. When using PCR fragments 1 and 2, these two fragments are introduced together into an expression system capable of expressing RNA. Another embodiment in which detected signals are normalized will be explained in detail in "1-3 Normalization of detected signals by internal control" described later.

RNAs are recovered from the expression system into which the aforementioned target-expressing molecule and the aforementioned subject nucleic acid molecule have been supplied and reverse-transcribed. A pair of primers which anneal to the sequences of the PCR primer annealing regions are supplied and an RT-PCR is performed to quantify the polynucleotides containing the target sequence. The expression system may be a cell-free expression system or a cell expression system. To perform the RT-PCR, cDNA is generated by using a reverse transcriptase and an oligo (dT) primer or random hexamers. DNAs are amplified by the PCR using the resulting cDNA as a template. The mRNA can be quantified by kinetically evaluating the amplification. Other conditions of the RT-PCR, such as temperatures and cycles, may be appropriately adjusted.

An RT-PCR is also performed for a system into which the subject nucleic acid molecule is not supplied, as well as the system into which the subject nucleic acid molecule has been supplied. Then, whether or not the RNA containing the target sequence has been cleaved is detected by comparison with the case in which the subject nucleic acid molecule is not supplied into the expression system. The system into which the subject nucleic acid molecule is not supplied also includes an embodiment in which a molecule (for example, a molecule similar to a subject nucleic acid molecule) that has been found not to have the RNAi activity on the target sequence is supplied for comparison.

The method for detecting an RNAi activity using the RT-PCR is not limited. For example, when the subject nucleic acid molecule sufficiently exerts an RNAi activity, which is often manifested as a delay of amplification of DNA containing the target sequence, the RNAi activity can be evaluated by measuring time-course of the amount of the amplified DNA. FIG. 4 shows an example of the evaluation method. FIG. 4 illustratively shows time-course of the amount of the amplified DNA. The horizontal axis indicates the number of PCR cycles and the vertical axis indicates the amount of DNA (the amount of PCR products) . In FIG. 4, the curve (I) shows the result from a system into which a subject nucleic acid molecule has not been supplied and the curve (II) shows the result from a system into which a molecule with an RNAi activity has been supplied. When the molecule with RNAi activity is supplied to the expression system, manifestation of the DNA amplification is delayed as indicated by the curve (II) in FIG. 4, which is different from the curve (I) in FIG. 4. This is because the subject nucleic acid molecule has cleaved an RNA transcribed from the target-expressing molecule, thereby decreasing the number of RT-PCR templates. Thus, the degree of this delay can be a marker to represent the degree of RNAi activity. In some cases, the amount of DNA is increased in spite of the fact that there is RNAi activity. This is because, depending on the degree of the activity, once a molecule that escaped RNAi comes to serve as a template, DNA can be easily amplified by PCR.

Alternatively, by being aware in advance of the time (or the number of PCR cycles) when a difference like that between (I) and (II) tends to appear, it is possible to evaluate RNAi activity based on the difference in amount of DNA measured only at that time, instead of periodically measuring.

### 1-2 Reporter method

Next, another embodiment is explained in which a reporter is used for a method to detect whether or not RNA containing a target sequence has been cleaved. In this reporter method, a sequence encoding an expression product detectable as a reporter is inserted into the target-expressing molecule at a location where the reporter can be transcribed as an mRNA integrated with the target sequence, and then expressions of the reporter is detected to evaluate whether or not the RNA has been cleaved. Also in the reporter method, the expression system may be a cell-free expression system or a cell expression system.

The reporter is not particularly limited as long as its expression can be detected. Such reporters include, for example, various kinds of fluorescence proteins, photoproteins, synthases, catabolic enzymes, resistance genes, etc. More specific examples of reporters include, for example, green fluorescent protein genes derived from Aequorea victoria, such as enhanced green fluorescent protein (EGFP); red fluorescent protein gene (DsRed) from Discoma sp.; near-infrared fluorescent protein gene (HcRed) from Heteractis crispa; firefly luciferase gene, Renilla luciferase gene, puromycin resistance genes, blasticidin S resistance gene, hygromycin resistance genes, β-galactosidase genes, chloramphenicol acetyltransferase genes, alkaline phosphatase genes, and mutants of these genes, etc. In addition, many other genes can preferably be used. The reporter is located in the target-expressing molecule where it can be transcribed as mRNA integrated with the target sequence. Accordingly, when an mRNA encoding the target sequence is cleaved, translation is inhibited for the reporter's sequence, leading to a phenomenon where the reporter's expression product is undetected or suppressed.

Detection of a reporter is appropriately selected depending on the type of the reporter. For example, when the reporter is a fluorescence protein or the like, the expression of the reporter can be detected by measuring the fluorescence intensity. When the reporter is a resistance gene, the expression of the reporter can be detected by assaying the resistance of cells into which the resistance gene has been introduced. When the reporter is a synthase or a catabolic enzyme, the expression of the reporter can be detected by quantifying products of the reaction catalyzed by these enzymes. In either case, quantitative or qualitative measurement is possible. In quantitative measurement, it is preferable to have the difference in amounts reflect the degree of RNAi activity.

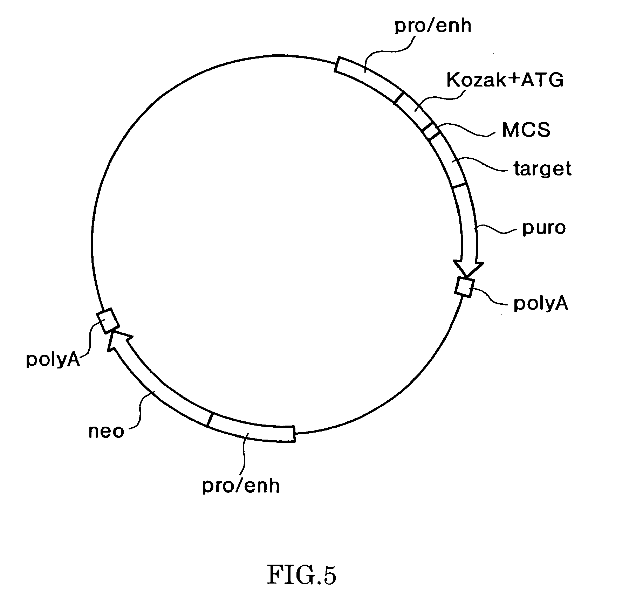

FIGs 5 to 7 show examples of construction of expression vectors as the target-expressing molecules into which a reporter is integrated. FIG. 5 is an expression vector into which a puromycin resistance gene ("puro") has been inserted as the reporter. The vector includes, from upstream to downstream, a promoter and enhancer "pro/enh", a Kozak sequence and ATG "Kozak+ATG" for allowing efficient translation, and a multiple cloning site ("MCS"), into which a target sequence ("target") and a puromycin resistance gene ("puro") are inserted. Further downstream of the puromycin resistance gene, a poly A signal ("poly A") is and, downstream thereof, a neomycin resistance gene ("neo").

The expression vector shown in FIG. 5 and a nucleic acid molecule are introduced into a cultured cell system to evaluate whether or not cells have puromycin resistance. When the subject nucleic acid molecule does not have an RNAi activity, the puromycin resistance gene is expressed giving puromycin resistance to the cells which can therefore grow in a puromycin-containing medium. In contrast, when the subject nucleic acid molecule has the RNAi activity, mRNAs are cleaved at the target sequence portion, and consequently a protein encoded by the puromycin resistance gene is untranslated. Therefore, the host cells do not have puromycin resistance, leading to a phenomenon where the cells cannot survive or their proliferation is suppressed. Based on such difference in cell growth, the presence or absence of RNAi activity can be determined. The expression vector shown in FIG. 5 contains a neomycin resistance gene ("neo").

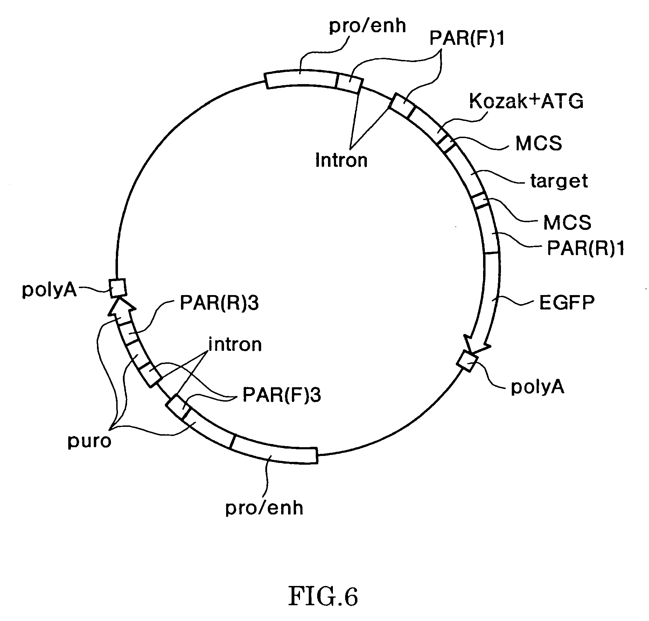

The expression vector shown in FIG. 6 contains an EGFP gene as a reporter. Specifically, an EGFP gene ("EGFP") has been inserted at the location where it is transcribed integrally with a target sequence. RNAi activity can be evaluated by detecting the amount of fluorescence from the EGFP. The amount of fluorescence can be measured according to conventional methods. Further, a puromycin resistance gene ("puro") is inserted as a resistance gene at a location under the control of a separate expression regulatory region from that for EGFP, allowing selection (concentration) of transfected cells by puromycin.

The expression vector shown in FIG. 6 contains a forward primer annealing region ("PAR (F)1") and a reverse primer annealing region ("PAR (R)1"), both of which flank an intron. Accordingly, using the expression vector shown in FIG. 6, detection by the above-mentioned RT-PCR can also be performed. The puromycin resistance gene ("puro") contains PAR(F)3 and PAR(R)3 flanking an intron. The RNA of the puromycin resistance gene can be quantified by using the primer annealing region within the puromycin resistance gene, thereby used as an internal control. Moreover, selection of the cells by using the puromycin resistance can increase the proportion of cells into which expression vectors etc. have been introduced. In other respects, the expression vector of FIG. 6 is the same as the expression vector of FIG. 5.

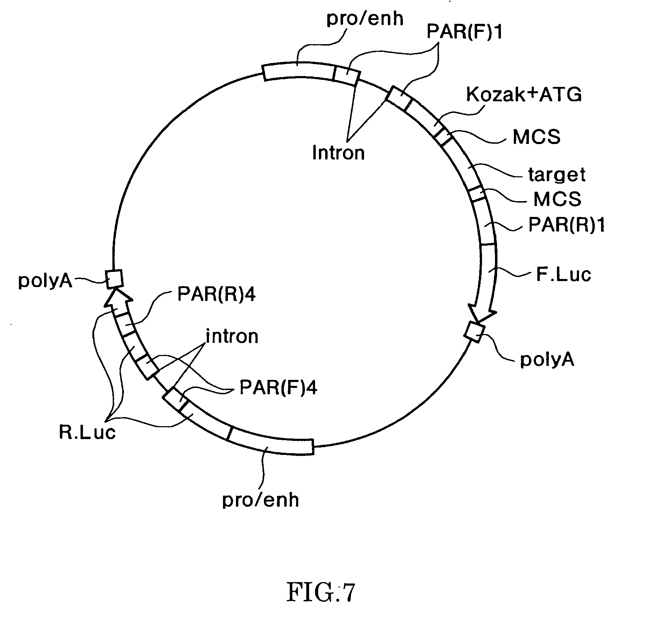

The expression vector shown in FIG. 7 contains a firefly luciferase gene ("F.Luc") as a reporter. Specifically, a firefly luciferase gene ("F.Luc") has been inserted at the location where it is transcribed integrally with a target sequence. In addition, a Renilla luciferase gene ("R.Luc") has been inserted at a separate location under the control of another expression regulatory region. RNAi activity can be determined by a luciferase assay method where luminescence is measured by a luminometer or the like. If the luminescence of the firefly luciferase is detected, it is determined that the RNAi activity is absent; and when the luminescence of the firefly luciferase is suppressed or undetected, it is determined that the RNAi activity is present. Luminescence of Renilla luciferase can be used as an internal control. Currently, the luciferase assay can serve as a simpler and more convenient assay and higher throughput evaluation method than RT-PCR. In other respects, the expression vector of FIG. 7 is the same as the expression vector of Fig. 5 or 6.

### 1-3 Normalization of detected signals by internal control

Another preferred embodiment according to the present invention is explained. In this embodiment, using an internal control, errors between test samples, specifically such as variations in transfection efficiency of a target-expressing molecule and a subject nucleic acid molecule, variations in the number of cells during recovery, variations in RNA yield after RNA extraction (RNA yield), variations in the amount of cDNAs added as PCR template, etc. can be corrected.

In the evaluation method according to the present invention, either in a cell expression system or a cell-free expression system, a target-expressing molecule and a subject nucleic acid molecule are supplied to an expression system and predetermined RNA is expressed from the target-expressing molecule. Meanwhile, as RNA is cleaved when RNAi activity is present, the presence of RNAi activity is judged by such phenomenon as that the amplification of predetermined DNA is suppressed, or the expression product is not detected. However, even when a target-expressing molecule etc. has not been appropriately supplied to the expression system or when the expression system is not appropriately functioning for some reason, there appears a phenomenon such as suppression of amplification of predetermined DNA or failure to detect expression product. Further, variations of the transfection efficiency of a target-expressing molecule and a subject nucleic acid molecule, the number of cells during recovery, the RNA yield after RNA extraction (RNA yield), or the amount of cDNAs added as PCR template, etc. can occur between samples. Thus, it is more preferred to provide means to correct such problems.

In this embodiment in which a normalization of detected signals is included, a control-supplying molecule containing a control sequence which can be transcribed into an mRNA that is not cleaved by the subject nucleic acid molecule and encodes a detectable expression product is supplied into an expression system into which the target-expressing molecule and the subject nucleic acid molecule are supplied.

The control-supplying molecule is a polynucleotide containing a control sequence, which encodes a detectable expression product. Examples of the expression product include, for example, an RNA, a polypeptide, a protein, etc. The control sequence is a sequence that is transcribed into an mRNA which at least is not cleaved by the subject nucleic acid molecule simultaneously supplied to the expression system. That is, in this embodiment using an introduction confirmation system, a molecule which does not undergo RNAi by a subject nucleic acid molecule is supplied. As long as the molecule meets the requirement that mRNA is not cleaved by a subject nucleic acid molecule, any molecule, for example, the reporter illustratively shown in "1-2" above, can be used. The detection method of an expression product is appropriately selected depending on the kind of the expression product.

A sequence that is not cleaved by a subject nucleic acid molecule may be a sequence confirmed in an experiment. For example, since the evaluation method according to the present invention can also easily detect a sequence in which a subject nucleic acid molecule does not exhibit RNAi activity, a sequence that has been confirmed in advance not to exhibit RNAi activity by the evaluation method according to the present invention can be used. Whether a subject nucleic acid molecule does not exhibit RNAi activity can be ascertained by the phenomena of amplification of DNA by RT-PCR and detection of an expression product. Accordingly, the problem of false positives does not occur and thus the presence or absence of RNAi activity can easily be determined.

Specific examples include, for example, the method using the combination of Figs. 2 and 3 or the like. In this method, the PCR fragment 2 shown in FIG. 3 is used as a control-supplying molecule. For example, separately from the PCR involving a target sequence by using PAR (F)1 and PAR (R)1, another PCR is performed using the combination of PAR (F)2 and PAR (R)2 involving the control sequence site. Then the quotient when the measured value of the PCR involving the target sequence is divided by the measured value of the PCR involving the control sequence site is calculated for each sample. In this way, errors between samples can be normalized, enabling more accurate comparison. Specifically, errors between samples, such as variations in transfection efficiency of a target-expressing molecule and a subject nucleic acid molecule, variations in the number of cells during recovery, variations in RNA yield after RNA extraction (RNA yield), and variations in the amount of cDNAs added as PCR template, etc. can be normalized.

The PCR fragment 2 has a neomycin resistance gene. In the example shown in FIG. 3, PCR primers are designed for PAR (F)2 and PAR (R)2 based on the sequence in the neomycin resistance gene as is.

Although a control-supplying molecule may be prepared and supplied separately from a target-expressing molecule as described above, as another embodiment, a control sequence may be integrated into target-expressing molecule. For this integration, the target-expressing molecule includes the control sequence and an expression regulatory region for expressing the control sequence. The control sequence can be similar to the control sequence contained in the above-mentioned control-supplying molecule.

When introducing a control sequence being incorporated into a target expressing molecule, the target sequence portion is always introduced into the expression system together with the control-supplying molecule. Therefore, incorporation of a control sequence into a target-expressing molecule provides an advantage that difference in transfection efficiency does not need to be considered between a control-supplying molecule and a target-expressing molecule. On the other hand, if a control-supplying molecule is not incorporated into a target-expressing molecule, the transfection efficiency of the target-expressing molecule can be enhanced because the transfection efficiency generally tends to become higher as the molecular size becomes smaller. Further, the target-expressing molecule composed of the PCR fragments shown in FIGs 2 and 3 is advantageous in that it can be simply and conveniently prepared by PCR without constructing a vector.

Specific examples of a target-expressing molecule include, for example, embodiments shown in FIGs 6 and 7. In the example shown in FIG. 6, the target-expressing molecule contains a puromycin resistance gene ("puro"), which contains an intron ("intron"), PAR (F)3, and PAR (R)3, and can be used as an internal control. In the example shown in FIG. 6, PAR (F)3 contains an intron. In other respects in the normalization technique using an internal control, the same explanation of normalization as in FIGs 2 and 3 mentioned above applies.

In the example shown in FIG. 7, the target-expressing molecule contains the Renilla luciferase gene ("R.Luc"), which serves as an internal control of a firefly luciferase gene. The Renilla luciferase gene incorporated into the target-expressing molecule contains PAR (F), PAR (R), and an intron. By computing "the value of luminescence of the firefly luciferase/the value of luminescence of the Renilla luciferase" relative activity of the luciferases can also be determined. Based on normalized values, more accurate comparison between data is possible.

### 1-4 Side effect confirmation system

siRNAs with RNAi activity, RNAi vectors expressing siRNAs, and the like can suppress expressions of the predetermined genes, and are therefore considered to be usable for gene therapy drugs etc. To enable such use, however, it is required that a certain specific siRNA should not produce side effects by acting on other genes in vivo.

Meanwhile, the sequence undergoing RNAi has the same nucleotide sequence as that of the siRNA. However, in some cases RNAi can occur when their sequences are only similar. It is therefore important to evaluate by what extent of similarity an RNAi may occur from the viewpoint of prevention of side effects mentioned above.

In this embodiment of the side effect confirmation system, subject nucleic acid molecule that has a different nucleotide sequence from the nucleotide sequence of the target sequence is used in the above-mentioned evaluation method according to the present invention. By venturing to use a nucleotide sequence different from the target sequence as an evaluation object, the extent of the RNAi activity that the subject nucleic acid molecule has can be defined.

### 2. Method for evaluating miRNA activity

In accordance with the present invention, there is further provided a method for evaluating an miRNA activity. The method for evaluating miRNA activity according to the present invention uses, as a subject nucleic acid molecule in the above-mentioned RNA activity evaluation method a molecule whose miRNA activity is to be evaluated. That is, the method for evaluating miRNA activity includes the steps of: supplying a target-expressing molecule, which is a polynucleotide containing at least a target sequence, an expression regulatory region regulating expression of an RNA containing the target sequence, a sequence encoding an expression product detectable as a reporter at the location where the reporter is transcribed as an mRNA integrated with the target sequence, and a subject nucleic acid molecule to be evaluated on whether or not the subject nucleic acid molecule has RNAi activity on an RNA containing the target sequence, into an expression system in which the target-expressing molecule is capable of expressing an RNA containing the target sequence; and detecting whether or not the RNA containing the target sequence has been cleaved.

miRNAs are known as noncoding RNAs of about 20 nucleotides long. Accordingly, as for a subject nucleic acid molecule, a noncoding RNA molecule of about 20 nucleotides long or a miRNA precursor having a hairpin structure is used. It should be noted that the target sequence-expressing molecule may be supplied into an expression system by using an expression vector expressing such RNA.

Since the miRNA is considered to act mainly as a translation suppressor, it is preferable to detect whether or not the expression of the protein encoded by the RNA containing a target sequence has been suppressed for more accurate evaluation of miRNAs. That is, a sequence encoding an expression product detectable as a reporter at the location where the reporter is transcribed as an mRNA integrated with the target sequence is incorporated into the target-expressing molecule. The technique for detecting miRNA activity can be performed by similar methods as explained in "1-2 Reporter method" in the method for evaluating RNAi activity described above. Whether an miRNA activity is present is judged by such a phenomenon as that the reporter is not detected or expression of the reporter is suppressed.

The expression system into which a target-expressing molecule etc. is supplied may be a cell-free expression system or a cell expression system. In addition, like the method for evaluating RNAi activity, also in the method for evaluating miRNA activity, the experimental system of normalization of detected signals by an internal control (refer to the above-mentioned "1-3") can be constructed. However, for the evaluation of miRNA activity, the control sequence contains a sequence whose expression is not suppressed by a subject nucleic acid molecule and encodes a detectable expression product. In addition, like the method for evaluating RNAi activity, a side effect confirmation system (refer to the above-mentioned "1-4") can be constructed.

### 3. Method for searching for RNA interference target nucleotide sequence

The method for searching for a target nucleotide sequence of RNA interference according to the present invention (hereinafter sometimes referred to as "the search method according to the present invention") is the method in which the nucleotide sequence of the target gene is searched for a nucleotide sequence that may trigger an RNA interference. Specifically, in the search method according to the present invention, the nucleotide sequence of the target gene for the RNA interference is searched for a sequence site that complies with the following rules (a) to (d):
(a) The nucleotide at the 3' end is adenine, thymine, or uracil.
(b) The nucleotide at the 5' end is guanine or cytosine.
(c) The seven-nucleotide sequence at the 3' end is rich in one or more nucleotide types selected from the group consisting of adenine, thymine, and uracil.
(d) The number of the nucleotides is a number that can trigger RNA interference without causing cytotoxicity.

A "gene" in the "target gene" described herein refers to the medium encoding genetic information. The "gene" is composed of substances encoding genetic information, such as DNA, RNA, DNA complexes, RNA complexes, etc. As for the genetic information, instead of the substances themselves but electronic data encoding the nucleotide sequence can be handled on a computer. The "target gene" may be one coding region or a plurality of coding regions. Alternatively, all the polynucleotides whose sequence has been determined may be targeted. When searches are to be performed as to a gene with a particular function, a nucleotide sequence that causes RNA interference specifically against the gene can be efficiently identified by using only that particular gene as a target. Since RNA interference is known as a phenomenon in which mRNA is interfered with and disrupted, search load can be reduced by selecting specific coding region(s). Also, a unit of transcriptional regions may be searched as the target gene. It should be noted that a nucleotide sequence that meets the aforementioned rules (a) to (d) is herein referred to as a "defined sequence". In the aforementioned rules, when the nucleotide sequence is a DNA sequence, thymine is adopted; when it is an RNA sequence, uracil is adopted.

The rule (c) specifies that the sequence near the 3' end is rich in nucleotide(s) selected from the group consisting of adenine, thymine, and uracil, i.e., as a marker to be used when specific searching is performed, it should be a sequence rich in nucleotide (s) selected from adenine, thymine, and uracil within the range of seven nucleotides from the 3' end.

In rule (c), a sequence "rich" in any nucleotide means that a particular nucleotide occurs at a high frequency in that sequence. To roughly show proportions, a 5 to 10-nucleotide sequence, preferably a 7-nucleotide sequence at the 3' end in a defined sequence contains one or more nucleotide types selected from the group consisting of adenine, thymine, and uracil by proportion (s) at least at 40% or higher, more preferably at 50% or higher. To be more specific, when a defined sequence of about 19 nucleotides is taken as an example, preferably at least 3 nucleotides or more, more preferably 4 nucleotides or more, particularly preferably 5 nucleotides or more among 7 nucleotides at the 3' end are one or more nucleotide types selected from the group consisting of adenine, thymine, and uracil.

The means of confirming the compliance with rule (c) is not limited to any specific one as long as it can confirm that preferably 3 nucleotides or more, more preferably 4 nucleotides or more, particularly preferably 5 nucleotides or more in 7 nucleotides at the 3' end are adenine, thymine, or uracil. To take as an example the case in which being rich is defined as the presence of three nucleotides or more of one or more nucleotide types selected from the group consisting of adenine, a thymine, and an uracil in the seven-nucleotide sequence of at the 3' end, the interpretation is as follows: each of the seven nucleotides is tested, one by one from the first from the 3' end, whether it is one of the three nucleotide types mentioned above. If three nucleotides of the same type occur when or before the seventh nucleotide is reached, the sequence can be judged to comply with rule (c). For example, if three nucleotides of the same type occur when the third nucleotide is reached, no further examination is required: i.e., when performing a search using rule (c), not all the seven nucleotides at the 3' end need necessarily to be tested. Conversely, if three nucleotides of the same type do not occur till the seventh nucleotide, the sequence is not rich in any nucleotides and therefore judged not to comply with rule (c).

It is known that adenine forms complementary hydrogen bonds with thymine or uracil in double-stranded polynucleotide. Further, three hydrogen bonding sites are formed in complementary hydrogen bonds between guanine and cytosine (G-C hydrogen bond) whereas two hydrogen bonding sites are present in complementary hydrogen bond between adenine a thymine, or uracil (A-(T/U) hydrogen bond). Generally, bonding strength of an A-(T/U) hydrogen bond is weaker than that of a G-C hydrogen bond.

Rule (d) specifies the number of nucleotides of a nucleotide sequence to be searched for: the number of nucleotides of the nucleotide sequence to be searched for is the number of nucleotides that can cause RNA interference to occur. It is known that a siRNA with too large a number of nucleotides have cytotoxicity, depending on the conditions such as the kind of organism. The upper limit of the number of nucleotides varies with, the kind of organism etc. in which RNA interference is to occur, but a preferred number of nucleotides of a single strand constituting a siRNA is 30 or smaller, regardless of the species. In the case of mammals, the number of nucleotides is preferably 24 or smaller and more preferably 22 or smaller. On the other hand, the lower limit of the number of nucleotides is not particularly limited as long it is a number that allows RNA interference to occur, but it is preferably 15 or larger, more preferably 18 or larger, and further preferably 20 or larger. As for the number of nucleotides of a single strand constituting a siRNA, it is particularly preferable to perform searches for 21.

As will be explained later again, an overhang is added to the 3' end of the defined sequence in a siRNA. A preferred number of nucleotides of the overhang is two. Therefore, the upper limit of the number of nucleotides of the defined sequence alone, excluding the overhang, is preferably 28 or smaller, more preferably 22 or smaller, and further preferably 20 or smaller; and the lower limit is preferably 13 or larger, more preferably 16 or larger, and still more preferably 18 or larger. The most preferred number of nucleotides of the defined sequence is 19. An RNAi target nucleotide sequence may be searched for in either with or without inclusion of an overhang.

A nucleotide sequence following the rules of the defined sequence causes RNA interference with an extremely high probability. Therefore, the search method according to the present invention makes it possible to identify a sequence that causes RNA interference with an extremely high probability and to simply and conveniently design a polynucleotide that causes RNA interference.

It should be noted that searches of defined sequences can be efficiently performed using a computer installed with a program for searching for sequence portions that follow the above-mentioned rules (a) to (c) based on the number of nucleotides defined.

### 4. Method for designing the nucleotide sequences of polynucleotides triggering RNA interference

In a method for designing the nucleotide sequences according to the present invention, a nucleotide sequence of a polynucleotide (a siRNA) that triggers RNA interference is designed based on the nucleotide sequence identified by the aforementioned search method. SiRNAs are composed mainly of RNA, but they may include mixed polynucleotides containing, in part, DNA. In the method for designing the nucleotide sequences according to the present invention, the nucleotide sequence of the target gene is searched for a sequence that follows the aforementioned rules (a) to (d), and a nucleotide sequence homologous to the nucleotide sequence retrieved is designed. Rules (a) to (d) and search techniques are as explained in the search method above.

A "homologous sequence" means an identical sequence, as well as a sequence containing mutations, such as deletions, substitutions, and/or additions, of that identical sequence to an extent that its function of causing RNAi is not lost. Depending on conditions, such as the kind and sequence of the target gene, the exemplary degree of acceptable mutations, is preferably 80% or higher, more preferably 90% or higher, and further preferably 95% or higher in homology. When calculating homology as the degree of acceptable mutations, it is desirable to compare between numerical values calculated by using a common search algorithm. The search algorithm is not particularly limited, but an algorithm suitable for searches of local sequences--more specifically, BLAST, SSearch, or the like--can preferably be used.

As mentioned above, some modifications are allowed to the sequence retrieved by the search, but the number of nucleotides of the nucleotide sequence to be designed is preferably the same as that of the sequence retrieved. When the number of nucleotides is the same, exemplary allowance of modifications for the nucleotides of the nucleotide sequence to be designed to match the nucleotides of the sequence retrieved is preferably 80% or more, more preferably at 90% or more, and particularly preferably at 95% or more. For example, when a 19-nucleotide sequence is to be designed, preferably 16 nucleotides or more, and more preferably 18 nucleotides or more, match the nucleotides of the nucleotide sequence retrieved. Further, when a sequence homologous to the nucleotide sequence retrieved is to be designed, the nucleotide at the 3' end of the nucleotide sequence retrieved is preferably the same as the nucleotide at the 3' end of the nucleotide sequence to be designed; and the nucleotide at the 5' end of the nucleotide sequence retrieved is preferably the same as the nucleotide at the 5' end of the nucleotide sequence to be designed.

Usually, an overhang is included in a siRNA molecule. An overhang refers to a single-stranded protrusion from the 3' end of each strand of a double-stranded siRNA molecule. A preferable number of nucleotides of an overhang, depends on the kind of the organism etc., and is most preferably two. Although the nucleotide sequence of an overhang is fundamentally arbitrary, a sequence such as the same nucleotide sequence as that of the target gene whose sequence is searched, TT, or UU is preferably used in some cases. By adding an overhang to the 3' end of the defined sequence designed, as described above, such that it is homologous to the nucleotide sequence retrieved, a sense strand constituting a siRNA is designed.

Alternatively, a siRNA can be designed based on a search performed with inclusion of an overhang into the defined sequence. The preferred number of nucleotides of an overhang is 2. Thus, for example, when a single strand constituting a siRNA composed of a 19-nucleotide defined sequence and a 2-nucleotide overhang is designed, a 21-nucleotide sequence may be searched for from the sequence of the target gene, and when a double-stranded siRNA is under consideration, a 23-nucleotide sequence may be searched for.

In the nucleotide sequence design method according to the present invention, the sequence of the target gene of interest is searched for a predetermined sequence, as described above. The origin of the subject to undergo the RNA interference may or may not match the origin of the target gene and a related species or the like as well. For example, when a gene isolated from a fist species is used as the target gene, an siRNA to be used for a second species related to the first species can also be designed. Further, by performing search for a common sequence of two or more species, e.g. mammals, followed by a search of this common sequence for the aforementioned defined sequence, a siRNA widely applicable to mammals can be designed. This is because a sequence common to a plurality of mammals is highly likely to be conserved in other mammals as well.

To prevent occurrence of RNA interference against other genes unrelated to the target gene, it is preferable to perform searches to check that no sequences identical or similar to the sequence designed are contained in the other genes. Sequences identical or similar to the sequence designed may be searched for by using general software programs enabling homology searches. By excluding such identical/similar sequences, a sequence that causes RNA interference specifically against the targeted gene alone can be designed.

The nucleotide sequence design method according to the present invention enables easy design of an RNA molecule that causes an RNA interference with high probability. RNA synthesis still requires effort, time, and cost, which can be significantly reduced by the nucleotide sequence design method according to the present invention.

### 5. Method for preparing double-stranded polynucleotides

The method for preparing double-stranded polynucleotides according to the present invention is a method for preparing double-stranded polynucleotides with a high probability of triggering RNA interference. The double-stranded polynucleotide is synthesized in accordance with the polynucleotide sequence design performed in accordance with the aforementioned nucleotide sequence design method. The preferred embodiment in sequence design is as explained in the aforementioned nucleotide sequence design method.

The double-stranded polynucleotide constitutes a double-stranded polynucleotide which triggers an RNA interference, as known as the siRNA. It should be noted that although double-stranded polynucleotides produced by the double-stranded polynucleotide preparation method according to the present invention are composed preferably of RNA, they may be mixed polynucleotides containing DNA in part. As described herein, a polynucleotide containing DNA in part is included in the concept of the siRNA. Further, studies by the inventors of the present invention have revealed that siRNAs tend to have asymmetry structurally and/or functionally. Considering the purpose of triggering RNA interference, a half at the 5' side of a sense strand and a half at the 3' side of an antisense strand are preferably composed of RNA.

The double-stranded polynucleotide is prepared such that one strand is formed from a sequence homologous to a defined sequence that is included in the nucleotide sequence of the target gene and that follows the rules (a) to (d) and an overhang added at the 3' end, and the other strand is formed from a sequence complementary to the nucleotide sequence homologous to the aforementioned defined sequence and an overhang added at the 3' end. The number of nucleotides of each strand including the overhang is 18 to 24, more preferably 20 to 22, and particularly preferably 21. The number of nucleotides of an overhang is preferably 2. An siRNA that has 21 nucleotides as a whole, including a two-nucleotide overhang, is favorable as an siRNA triggering RNA interference with a high probability without causing cytotoxicity in mammals.

The RNA may be synthesized, for example, by chemical synthesis, or in accordance with conventional techniques of biotechnology or the like. To give one example, RNA can be synthesized by synthesizing a DNA strand having a predetermined sequence, synthesizing a single stranded RNA with a transcriptase by using the aforementioned DNA strand, and then converting the single-stranded RNA into a double-stranded RNA.

The preferred embodiment of the polynucleotide obtained by the above-mentioned method for preparing a double-stranded polynucleotide is illustratively a double-stranded polynucleotide which is prepared, based on a search of the nucleotide sequence of the RNAi target gene for a 13- to 28-nucleotide sequence site that follows the aforementioned rules (a) to (d), such that one strand is formed from a sequence homologous to the defined sequence that is included in the nucleotide sequence of the target gene and that follows rules (a) to (d) and an overhang added at the 3' end, and the other strand is formed from a sequence complementary to the nucleotide sequence homologous to the aforementioned defined sequence and an overhang added at the 3' end, with the number of the nucleotides of each strand being 5 to 30. This polynucleotide is a polynucleotide that is highly likely to trigger an RNA interference.

Alternatively, an expression vector capable of expressing an siRNA can also be prepared. An expression vector that expresses a sequence containing the defined sequence can be placed under the condition of a cell-free system or a cell system in which the expression can occur to supply the specified siRNA.

Conventionally, siRNA designing has relied on the experiences and the intuitions of experimenters, often requiring repeated trials and errors. However, the double-stranded polynucleotide preparation method according to the present invention enables preparation of a double-stranded polynucleotides that triggers RNA interference with a high probability. The above-mentioned target nucleotide sequence search method, the sequence design method, and/or the double-stranded polynucleotide preparation method can significantly reduce efforts, time, and costs required for various tests, productions, etc. using the RNA interference, whereby significantly simplifying and facilitating various tests, researches, developments, productions, etc. using the RNA interference, such as gene analysis, search for drug discovery targets, drug discovery, gene therapy, study of the difference among organism species etc.

### 6. Method for suppressing gene expression

The method for suppressing gene expression according to the present invention includes a step of searching for a sequence to retrieve a certain nucleotide sequence; steps of designing a nucleotide sequence of a siRNA based on the retrieved nucleotide sequence and then synthesizing an siRNA; and a step of introducing the resulting siRNA into an expression system including the target gene.

The step of searching for a sequence to retrieve a certain nucleotide sequence is performed in accordance with the aforementioned method for searching for a nucleotide sequence of the target gene of RNA interference. The preferred embodiments are also as described above. The steps of designing the nucleotide sequence of a siRNA based on the nucleotide sequence retrieved and then synthesizing the siRNA can be performed also in accordance with the aforementioned method for searching for a nucleotide sequence of the target gene of RNA interference and the method for preparing double-stranded polynucleotide. The preferred embodiments are the same.

The resulting double-stranded polynucleotide is added to a target gene expression system to suppress the expression of the target gene. The target gene expression system is a system in which the target gene is being expressed. More specifically, it is a system including a reaction system in which mRNA of the target gene is formed. Target gene expression systems include in vitro systems and in vivo systems. For the target gene expression system, a cell-free system can be used, as well as cultured cells, cultured tissue, an organism, etc. The target gene whose expression is to be suppressed (suppression target gene) may or may not be a gene of an organism species that matches the origin of the sequence identified by searching. However, the closer the origin of the gene searched for and that of the target gene suppressed are, the more specifically and efficiently can a certain gene be suppressed.

"Introducing into a target gene expression system" means to make a subject of the introduction incorporated in the expression system. Specific examples include the technique of transfecting cultured cells with double-stranded polynucleotides having the target gene so that the polynucleotides are incorporated into cells; the technique of constructing an expression vector having a nucleotide sequence composed of a defined sequence and an overhang and introducing it into cells having the target gene, etc.

According to the present method for suppressing gene expression, polynucleotide triggering RNA interference can be efficiently obtained, thereby the gene expression can be efficiently suppressed in a simple and convenient manner.

The present invention will be described in more detail by way of examples of the present invention as follows, which should not be construed as limiting the present invention.

### EXAMPLES

### Example 1

### 1. Construction of target expression vector pTREC

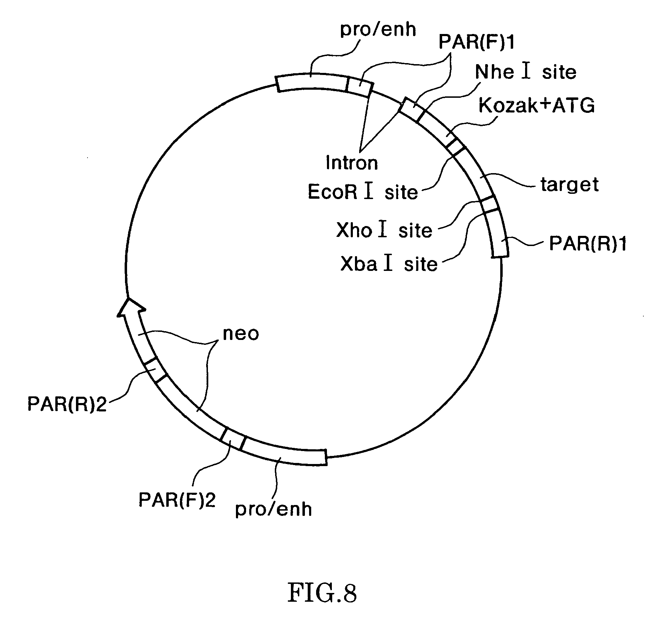

A target mRNA sequence was constructed downstream of the CMV enhancer/promoter of pCI-neo (GenBank Accession No. U47120; manufactured by Promega Corporation) (FIG. 8). A double-stranded oligomer having a Kozak sequence ("Kozak"), a 23-bp sequence to be targeted ("target"), and recognition sequences of restriction enzymes (NheI, EcoRI, and XhoI) for recombination was synthesized as shown below. The double-stranded oligomer is composed of the sequence shown in SEQ ID NO: 1 in the sequence listing and a complementary sequence thereof. The target expression vector pTREC was constructed by inserting the synthesized double-stranded oligomer into between the NheI and XbaI sites of pCI-neo (FIG. 8). As for the intron, an intron site derived from β-globin having been integrated into pCI-neo was used.
5'-gctagccaccatggaattcacgcgtctcgagtctaga-3' (SEQ ID NO: 1)

### 2. Effect of target mRNA detection primer

### (1) Transfection into cultured cells

HeLa cells were plated at 0.2 to 0.3 x 10⁶ per well in 24-well plates. After one day, the cells were transfected with 0.5 µg of the pTREC vector by using Lipofectamine 2000 (manufactured by Invitrogen) in accordance with the instruction manual.

### (2) Recovery of cells and quantification of mRNA

After one day of the transfection, cells were recovered and total RNAs were extracted using Trizol (manufactured by Invitrogen). A cDNA synthesis reaction was performed by reverse-transcribing 100 ng of the extracted RNA by using oligo (dT) as primer with SuperScript II RT (manufactured by Invitrogen). As a control, RNA to which reverse transcriptase was not added was prepared. Using 1/320 volume of the resulting cDNA as a PCR template, quantitative PCR was performed with SYBR Green PCR Master Mix (manufactured by Applied Biosystems) in a 50 µl reaction system to quantify the target mRNA (referred to as mRNA (T)) and the mRNA derived from the neomycin resistance gene on pTREC (referred to as mRNA (C)) as an internal control. A real-time PCR monitoring system ABI PRIZM 7000 (manufactured by Applied Biosystems) was used for the quantitative PCR. The primer pair T (SEQ ID NOs: 2 and 3 in the sequence listing) was used for the quantification of mRNA (T) ; and the primer pair C (SEQ ID NOs: 4 and 5 in the sequence listing) was used for the quantification of mRNA (C).

Primer pair T:
aggcactgggcaggtgtc (SEQ ID NO: 2)
tgctcgaagcattaaccctcacta (SEQ ID NO: 3)

Primer pair C:
atcaggatgatctggacgaag (SEQ ID NO: 4)
ctcttcagcaatatcacggt (SEQ ID NO 5)

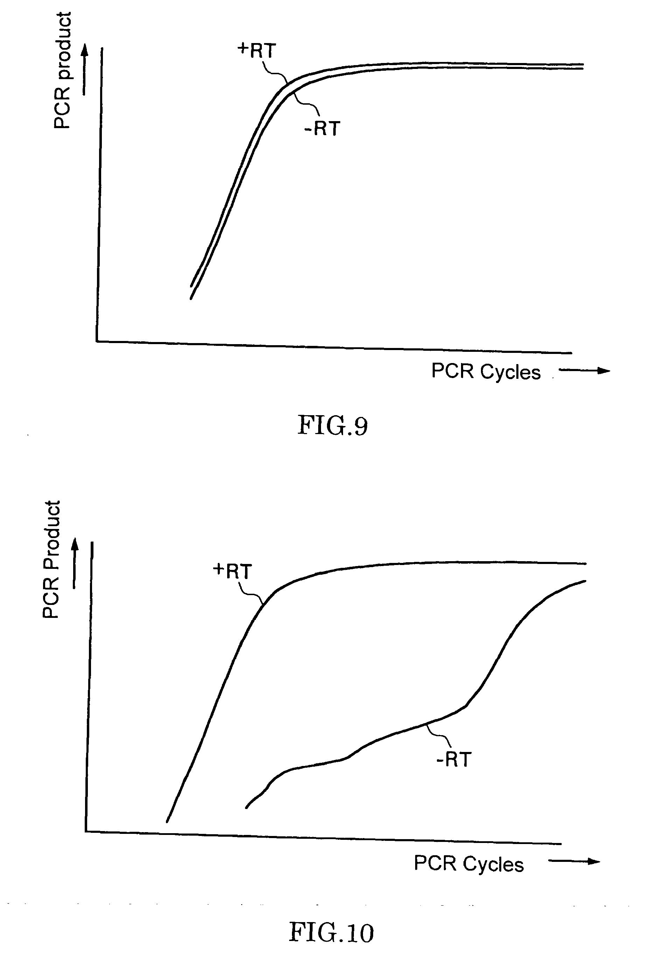

The results of the PCR are shown in FIGs. 9 and 10. FIGs. 9 and 10 show graphs of the amounts of respective PCR products on the vertical axis and the numbers of PCR cycles on the horizontal axis. In the case of the neomycin resistance gene, there was only a small difference in the amounts of the resulting PCR products between cDNA synthesized with reverse transcriptase (+RT) and the control (-RT) to which reverse transcriptase was not added (FIG. 9). This indicates that not only the cDNA but also the vector remaining in cells served as PCR template and amplified. In contrast, for target mRNA sequence, there was a large difference between the presence (+RT) and the absence (-RT) of the reverse transcriptase (FIG. 10). This result suggests that, since one of the primer T is designed to flank an intron, the cDNA derived from mRNA from which the introns were spliced out is efficiently amplified, whereas the remaining vector having introns was less likely to serve as template.

### 3. Suppression of target mRNA expression by siRNAs

### (1) Cloning of the evaluation sequence into a target expression vector

Sequences corresponding to 812-834 and 35-57 of the coding regions of the human vimentin (VIM) gene (Ref Seq ID: NM_003380) were used as subjects of evaluation. Synthetic oligonucleotides (evaluation sequence fragments) of these sequences and the following SEQ ID NOs: 6 and 7 in the sequence listing that have recognition sequence for EcoRI and XhoI were prepared. Evaluation sequence VIM-35 (corresponding to 35-57 of VIM) 5'-gaattcgcaggatgttcggcggcccgggcctcgag-3' (SEQ ID NO: 6) Evaluation sequence VIM-812 (corresponding to 812-834 of VIM) 5'-gaattcacgtacgtcagcaatatgaaagtctcgag-3' (SEQ ID NO: 7)

pTREC-VIM35 and pTREC-VIM812 were constructed by cloning resulting evaluation sequence fragments into pTREC between the EcoRI and XhoI sites using the EchoRI and XhoI sites that flank the fragments.

### (2) Preparation of siRNAs

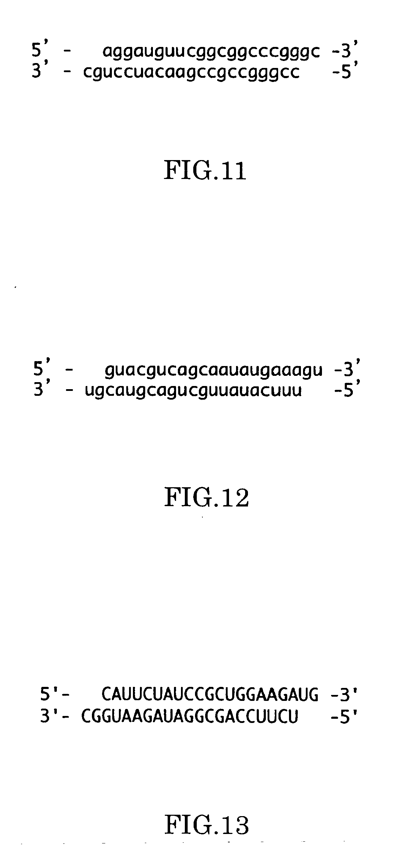

siRNA fragments corresponding to the evaluation sequence VIM-35 (SEQ ID NO: 8 in the sequence listing; FIG. 11), the evaluation sequence VIM-812 (SEQ ID NO 9; FIG. 12), and the control sequence (siControl, SEQ ID NO: 10; FIG. 13) were synthesized and subjected to annealing. Each of the following siRNA sequences is designed to contain an overhang sequence at the 3' end.
siVIM-35 5'-aggauguucggcggcccgggc-3' (SEQ ID NO: 8)
siVIM-812 5'-guacgucagcaauaugaaagu-3' (SEQ ID NO: 9)

As the control, a siRNA directed against the luciferase gene was used.
siControl 5'-cauucuauccgcuggaagaug-3' (SEQ ID NO: 10)

### (3) Transfection into cultured cells

HeLa cells were plated at 0.2 to 0.3 x 10⁶ per well in 24-well plates. After one day, the cells were cotransfected with 0.5 µg of TREC-VIM35 or pTREC-VIM812 and siRNAs corresponding to respective VIM-derived sequences (siVIM-35 and siVIM-812; final concentration 100 nM each) by using Lipofectamine 2000 (manufactured by Invitrogen) in accordance with the instruction manual. Control cells were cotransfected with 0.5 µg of TREC-VIM35 or pTREC-VIM812 and a siRNA (siControl; final-concentration 100 nM) directed against the luciferase gene.

### (4) Recovery of cells and quantification of mRNA

After one day of the transfection, cells were recovered and total RNAs were extracted using Trizol (manufactured by Invitrogen) . A cDNA synthesis reaction was performed by reverse-transcribing 100 ng of the extracted RNA by using oligo (dT) as primer with SuperScript II RT (manufactured by Invitrogen). As a control, RNA to which reverse transcriptase was not added was prepared. Using 1/320 volume of the resulting cDNA as a PCR template, quantitative PCR was performed with SYBR Green PCR Master Mix (manufactured by Applied Biosystems) in a 50 µl reaction system to quantify the mRNA carrying a VIM-derived sequence to be evaluated (referred to as mRNA (T)) and the mRNA derived from the neomycin resistance gene on pTREC (referred to as mRNA (C)) as the internal control.

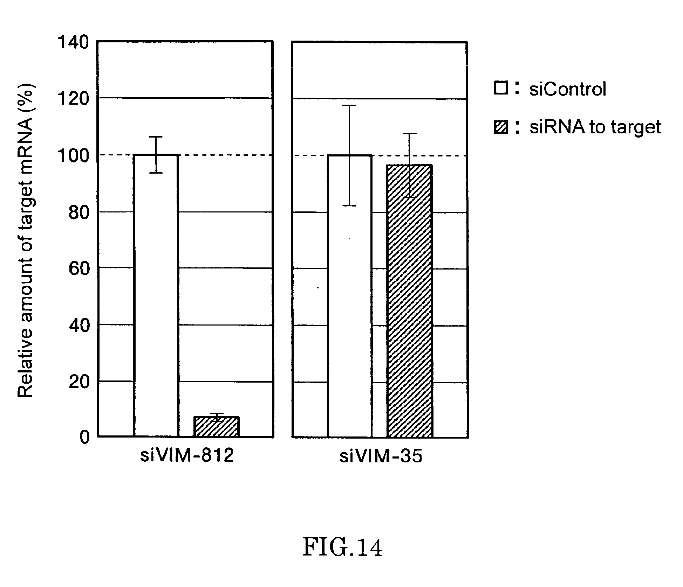

The real-time PCR monitoring system ABI PRIZM 7000 (manufactured by Applied Biosystems) was used for the quantitative PCR. The primer pair T (SEQ ID NOs: 2 and 3 in the sequence listing) was used for the quantification of mRNA (T); and the primer pair C ( SEQ ID NOs: 4 and 5 in the sequence listing) was used for the quantification of mRNA (C). The results were presented in a graph with the ratio of the values of each mRNA obtained on the vertical axis (relative amount of target mRNA (%)) (FIG. 14).

In the case of control cells, the siRNA directed against the luciferase gene exerted no effect on the target mRNA, and thus the T/C ratio was almost 1. For the VIM-812 siRNA, the T/C ratio was remarkably smaller. This is because the VIM-812 siRNA had cleaved mRNAs whose sequences are homologous to its own sequence, indicating the VIM-812 siRNA has the RNAi effect. In contrast, for the VIM-35 siRNA, the T/C ratio was almost the same as that for the control, indicating that the VIM-35 siRNA sequence has very little RNAi effect.

### Example 2

### 1. Suppression of endogenous vimentin expression by siRNAs

### (1) Transfection into cultured cells

HeLa cells were plated at 0.2 to 0.3 x 10⁶ per well in 24-well plates. After one day, the cells were cotransfected with the siRNA (siVIM-35 or siVIM-812) directed against VIM or the control siRNA (siControl), 100 nM each, and 0.5 µg of pEGFP (manufactured by Clontech) as the control of transfection efficiency by using Lipofectamine 2000 (manufactured by Invitrogen) in accordance with the instruction manual. pEGFP contains EGFP.

### (2) Measurement of endogenous vimentin mRNA

After three days of the transfection, cells were recovered and total RNAs were extracted using Trizol (manufactured by Invitrogen). A cDNA synthesis reaction was performed by reverse-transcribing 100 ng of the extracted RNA by using oligo (dT) as primer with SuperScript II RT (manufactured by Invitrogen). Using the resulting cDNA products as a template, PCR was performed with the primers VIM-F 3-84 and VIM-R 3-274 (SEQ ID NOs: 11 and 12) directed against vimentin. VIM-F3-84: gagctacgtgactacgtcca (SEQ ID NO: 11) VIM-R 3-274: gttcttgaactcggtgttgat (SEQ ID NO: 12)

Further, a PCR was performed with primers ACTB-F 2-481 and ACT B-R 2-664 (SEQ ID NOs 13 and 14) directed against β-actin and the quantitative values of β-actin were normalized between the samples to evaluate the expression level of vimentin.
ACTB-F 2-481: cacactgtgcccatctacga (SEQ ID NO: 13)
ACTB-R 2-664: gccatctcttgctcgaagtc (SEQ ID NO: 14)

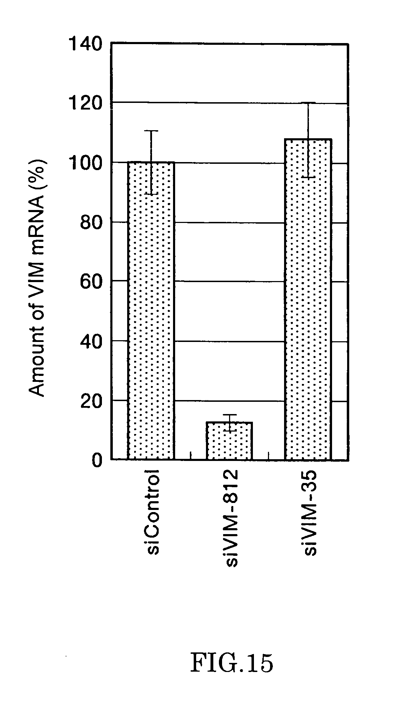

The results are shown in FIG. 15. FIG. 15 indicates to what extent VIM mRNA was decreased when the siRNAs against VIM was introduced by comparison to the value when siControl was introduced (i.e., a siRNA whose sequence is irrelevant to that of the target) as 100%. siVIM-812 was able to efficiently suppress VIM mRNA, whereas siVIM-35 exhibited very little RNAi effect.

### (3) Antibody staining of cells

Three days after transfection, cells were fixed with 3% paraformaldehyde and blocked by a conventional method. Then, a rabbit anti-vimentin antibody (α-VIM) or a rabbit ant-Yes antibody (α-Yes) as an internal control was added and reacted at room temperature. The cell surface was washed with phosphate-buffered saline (PBS). A fluorescent-labeled anti-rabbit IgG antibody was added as a second antibody and reacted at room temperature. After the cell surface was washed with PBS, the cells were observed under a fluorescence microscope.

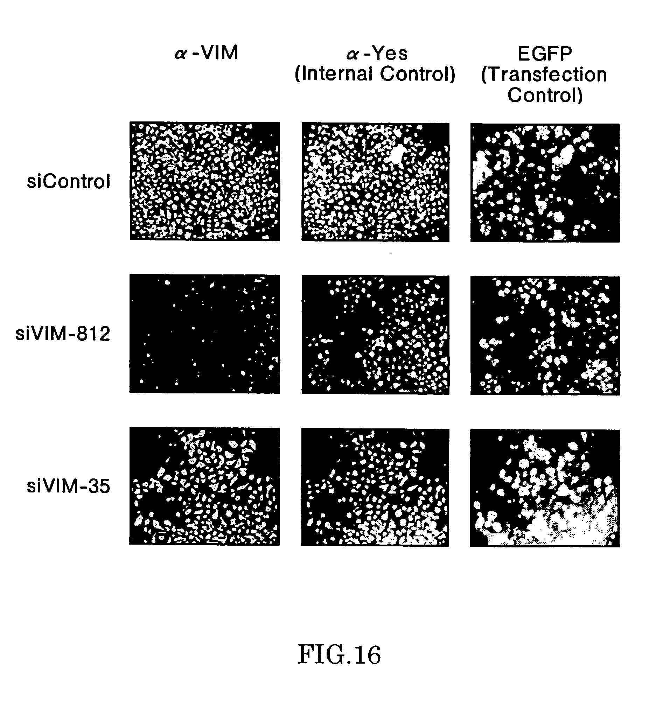

The result of the fluorescence microscopic observation is shown in FIG. 16. In each of the nine frames in FIG. 16, a white area indicates where fluorescence has emerged. In all cells, the same level of expression of EGFP and Yes were confirmed. In cells into which siControl and siVIM-35 had been introduced, fluorescence by vimentin antibody staining was observed, and thus the presence of endogenous vimentin was confirmed. In contrast, in cells into which siVIM-812 had been introduced, fluorescence was notably weaker, as compared with the cells in which siControl and siVIM-35 had been introduced. These results indicate that endogenous vimentin mRNA has undergone interference by siVIM-812, resulting in a decrease in vimentin expression level. Thus, it is revealed that siVIM-812 exhibits RNAi effect on endogenous vimentin mRNA as well.

The results obtained in the assay system according to the present invention (Example 1) closely correlated with the results of the actual application of each siRNA to an endogenous gene (Example 2), suggesting that the assay system is an effective system as the method for evaluating RNAi activity of any siRNAs.

### INDUSTRIAL APPLICABILITY

The present invention can be suitably used for biotechnological experiments, researches, and developments, and, further, pharmaceutical developments, etc.

### SEQUENCE LISTING FREE TEXT

SEQ ID NO 1: Oligomer containing restriction sites for NheI, EcoRI, and XhoI,
SEQ ID NO 2: PCR primer T
SEQ ID NO 3: PCR primer T
SEQ ID NO 4: PCR primer C
SEQ ID NO 5: PCR primer C
SEQ ID NO 6: Target sequence VIM35
SEQ ID NO 7: Target sequence VIM812
SEQ ID NO 8: siRNA to be evaluated, siVIM-35
SEQ ID NO 9: siRNA to be evaluated, siVIM-812
SEQ ID NO 10: Control siRNA, siControl
SEQ ID NO 11: PCR primer VIP-F3-84,
SEQ ID NO 12: PCR primer VIP-R3-274
SEQ ID NO 13: PCR primer ACTB-F2-481
SEQ ID NO 14: PCR primer ACTB-R2-664

## Claims

1. A method for evaluating RNAi activity, comprising the steps of:
supplying a target-expressing molecule, which is a polynucleotide containing at least a target sequence and an expression regulatory region which regulates an expression of an RNA containing said target sequence, and
a subject nucleic acid molecule to be evaluated on whether or not the subject nucleic acid molecule has an RNAi activity on said RNA containing the target sequence
into an expression system in which said target-expressing molecule is capable of expressing said RNA containing the target sequence; and
detecting whether or not said RNA containing said target sequence has been cleaved.

2. The evaluation method for RNAi activity of claim 1, wherein the target-expressing molecule comprises, both upstream and downstream of the target sequence, a PCR primer annealing region containing a sequence to which a PCR primer can anneal, said method comprising the steps of:
quantifying said RNA containing the target sequence generated by supplying said target-expressing molecule and said subject nucleic acid molecule into said expression system by RT-PCR using a pair of primers annealing to the sequence in the PCR primer annealing region; and
detecting whether or not said RNA containing the target sequence has been cleaved by comparing with the case in which said subject nucleic acid molecule is not supplied into said expression system.

3. The method for evaluating RNAi activity of claim 2, wherein an intron is flanked within at least one of said PCR primer annealing regions present both upstream and downstream of the target sequence, and said expression system is an expression system capable of splicing an RNA.

4. The method for evaluating RNAi activity of claim 1, wherein said target-expressing molecule comprises a sequence encoding an expression product detectable as a reporter at a location whereby said reporter is to be transcribed as an mRNA integrated with the target sequence, the method comprising a step of detecting the expression of said reporter to detect whether or not the RNA has been cleaved.

5. The method for evaluating RNAi activity of any one of claims 1 to 4, wherein the expression system is a cell-free expression system or a cell expression system.

6. The method for evaluating RNAi activity of any one of claims 1 to 5, comprising a step of supplying into said expression system into which said target-expressing molecule and said subject nucleic acid molecule are supplied a control-supplying molecule that comprising a control sequence which comprises a sequence to be transcribed into an mRNA that is not cleaved by said subject nucleic acid molecule and encodes a detectable expression product.

7. The method for evaluating RNAi activity of any one of claims 1 to 5, wherein a control sequence which comprises a sequence to be transcribed into an mRNA that is not cleaved by said subject nucleic acid molecule and encodes a detectable expression product is integrated into said target-expressing molecule.

8. The method for evaluating RNAi activity of any one of claims 1 to 7, wherein said target sequence comprises a nucleotide sequence different from the nucleotide sequence that said subject nucleic acid molecule comprises.

9. A method for evaluating miRNA activity, comprising the steps of:
supplying a target-expressing molecule, which is a polynucleotide comprising:
at least a target sequence, an expression regulatory region regulating an expression of an RNA containing said target sequence, and a sequence encoding an expression product detectable as a reporter at a location whereby said reporter is to be transcribed as an mRNA integrated with said target sequence; and
a subject nucleic acid molecule to be evaluated on whether or not said subject nucleic acid molecule has an RNAi activity on an RNA containing said target sequence
into an expression system in which said target-expressing molecule is capable of expressing said RNA containing the target sequence; and
detecting whether or not said RNA containing the target sequence has been cleaved.

10. The method for evaluating miRNA activity of claim 9, wherein said expression system is a cell-free expression system or a cell expression system.

11. The method for evaluating miRNA activity of claim 9 or 10, comprising a step of supplying into said expression system into which said target-expressing molecule and said subject nucleic acid molecule are supplied a control-supplying molecule which comprises a control sequence comprising a sequence whose expression is not suppressed by said subject nucleic acid molecule and encodes a detectable expression product.

12. The method for evaluating miRNA activity of claim 11, wherein said control-supplying molecule is integrated into said target-expressing molecule.

13. The method for evaluating miRNA activity of any one of claims 9 to 12, wherein said target sequence comprises a nucleotide sequenced different from the nucleotide sequence that said subject nucleic acid molecule comprises.


SEQUENCE LISTING

<110>  NAITO, Yuki; TEI, Kumiko; ZENNO, Shuhei; SAIGO, Kaoru;
CellFree Sciences Co., Ltd.

<120>  Method of evaluation for RNAi activities and miRNA activities

<130>  PSSA-15490

<160>  14

<170>  PatentIn version 3.1

<210>  1
<211>  37
<212>  DNA
<213>  Artificial

<220>
<223> Inventor: NAITO, Yuki
<220>
<223> Inventor: UI-TEI, Kumiko
<220>
<223> Inventor: ZENNO, Shuhei
<220>
<223> Inventor: SAIGO, Kaoru

<220>
<223>  oligomer including Nhe I site, EcoRI site and Xho I site

<400>  1
gctagccacc atggaattca cgcgtctcga gtctaga
37

<210>  2
<211>  18
<212>  DNA
<213>  Artificial

<220>
<223>  PCR primer T

<400>  2
aggcactggg caggtgtc                                                    18

<210>  3

<211>  24
<212>  DNA
<213>  Artificial

<220>
<223>  PCR primer T

<400>  3
tgctcgaagc attaaccctc acta                                              24


<210>  4
<211>  21
<212>  DNA
<213>  Artificial

<220>
<223>  PCR primer C

<400>  4
atcaggatga tctggacgaa g                                                 21


<210>  5
<211>  21
<212>  DNA
<213>  Artificial

<220>
<223>  PCR primer C

<400>  5
ctcttcagca atatcacggg t                                                 21


<210>  6
<211>  35
<212>  DNA
<213>  Artificial

<220>
<223>  target sequence VIM35

<400>  6
gaattcgcag gatgttcggc ggcccgggcc tcgag                                  35


<210>  7
<211>  35

<212>  DNA
<213>  Artificial

<220>
<223>  target sequence VIM812

<400>  7
gaattcacgt acgtcagcaa tatgaaagtc tcgag                                35


<210>  8
<211>  21
<212>  RNA
<213>  Artificial

<220>
<223>  siRNA as evaluation subject; siVIM35

<400>  8
aggauguucg gcggcccggg c                                               21


<210>  9
<211>  21
<212>  RNA
<213>  Artificial

<220>
<223>  siRNA as evaluation subject; siVIM812

<400>  9
guacgucagc aauaugaaag u                                               21


<210>  10
<211>  21
<212>  RNA
<213>  Artificial

<220>
<223>  Control siRNA; siControl

<400>  10
cauucuaucc gcuggaagau g                                               21


<210>  11
<211>  20
<212>  DNA

<213>  Artificial

<220>
<223>  PCR primer VIM-F3-84

<400>  11
gagctacgtg actacgtcca                                                    20


<210>  12
<211>  21
<212>  DNA
<213>  Artificial

<220>
<223>  PCR primer VIM-R3-274

<400>  12
gttcttgaac tcggtgttga t                                                  21


<210>  13
<211>  20
<212>  DNA
<213>  Artificial

<220>
<223>  PCR primer ACTB-F2-481

<400>  13
cacactgtgc ccatctacga                                                    20


<210>  14
<211>  20
<212>  DNA
<213>  Artificial

<220>
<223>  PCR primer ACTB-R2-664

<400>  14
gccatctctt gctcgaagtc                                                    20